# EUROPEAN PATENT SPECIFICATION

(11) **EP 0 174 336 B1**
(45) Date of publication and mention of the grant of the patent: **20.01.1993**
(21) Application number: 85901128.0
(22) Date of filing: 22.02.1985
(51) Int. Cl.: A61M 16/00, A61M 15/00, A61M 16/10

(54) **BREATHING APPARATUS**
ATMUNGSVORRICHTUNG
APPAREIL RESPIRATOIRE

(30) Priority: 01.03.1984 SE 8401143
(43) Date of publication of application: 19.03.1986
(73) Proprietor: LINDHOLM, Nore, F-67390 Artolsheim (FR)
(72) Inventor: LINDHOLM, Nore, F-67390 Artolsheim (FR)
(74) Representative: Rostovanyi, Peter
(86) International application number: SE8500088
(87) International publication number: WO8503880

(56) References cited:
- CA-A- 1 080 579
- SE-B-82 063 207
- US-A- 4 261 354
- US-A- 4 262 666

## Description

An arrangement in instruments intended to be held partially or totally in the mouth of an individual, for enabling swallowing reflexes to function substantially normally and/or to facilitate articulation, without needing to remove the object manually from the mouth.

### Technical field

The present invention relates to an arrengement in instruments of the kind which are intended to be held partially or totally in the mouth of an individual, for enabling swallowing reflexes of the individual to function substantially normally and/or to facilitate articulation without it being necessary to remove the instrument from the mouth manually. Examples of such instruments are breathing devices incorporating a heat-exchange function for minimizing the dead-space volume, as described in WO-A-84/01720, mouth-carried heat-exchange devices for preventing the build-up of condensation on automobile windows (CA-A- 1 080 579), and suction devices used in dentistry work, etc.

The fact that speech and swallowing functions are impaired when holding a relatively large instrument or object in the mouth is generally known by all. When wishing to speak or needing to swallow it is often necessary to remove the object before being able to do so. At times, however, the fact that the object must be removed manually may constitute a problem. For example, when the object concerned is a mouth-carried anti-mist heat-exchange device worn by the driver of an automobile, it may be inconvenient for the driver to release the controls of the vehicle, gear lever, driving wheel etc., in order to remove the device.

Another example of cases where to facilitate speech and swallowing functions the manual removal of mouth-carried instruments of the aforesaid kind may be problematic is found in respect of handicapped or paralysed people who breath through the mouth and who wear a device of the kind described in the aforesaid Swedish Patent Application for improving their physical working capacity, e.g. by minimizing the dead-space volume.

### Disclosure of the Invention

An object of the invention is to provide in such objects or instruments as those intended to be held totally or partially in the mouth an arrangement which enables the wearer to speak normally and to swallow comfortably while still holding the object in his/her mouth.

A further object is to provide in such instruments or objects an arrangement which enables the position of the object in the mouth to be changed in a manner to permit the wearer to speak and to swallow normally.

Accordingly this invention consists in a breathing device which is intended to be placed and held in the mouth of a person and which comprises a tubular casing and positioned therein a plurality of parallel channels open at each end, the casing having a proximal open end opposite to a distal open end, the proximal end of the casing being concave and being provided thereon with two outwardly and laterally jutting gripping flanges extending in opposite directions, the gripping flanges extending at such an angle from the casing and being so dimensioned as:
- firstly to enable the device to be moved by the facial muscles and/or the tongue from an operative position, in which the flanges are located inwardly of the jaws or teeth relatively far back in the oral cavity, to a speaking or swallowing position, in which the device is held forwardly of the jaws or teeth, and to be returned to the operative position by the facial muscles and alternatively also the teeth;
- secondly to enable the person to hold the device firmly in the speaking or swallowing position by an interaction between the gripping flanges and the soft parts of the mouth, such as the cheeks or lips;
- and thirdly to permit the jaws to be substantially closed when the device is in the speaking or swallowing position.

A highly complicated system of muscle movements is required in order to enable saliva to pass from the oral cavity to the oesophagus without entering the nasal cavity or the trachea. In order for a person to swallow effectively and without discomfort it is necessary for all passages irrelevant to this function to be closed at the moment of passing saliva through the throat to the oesophagus with the aid of the relevant muscles. The swallowing function of an individual can be divided into a number of part functions. The present description is restricted solely to those functions which concern the invention.

If any part of the swallowing function is excluded for some reason or other, then the person concerned totally loses his or her ability to swallow and will dribble or slobber as a result thereof. All of the aforesaid part functions are associated totally or partially with reflex movements. The two part functions described below, however, can be controlled or influenced by instinctive action resulting from the will to carry out such part functions.

Part function 1 explains the inability to swallow with the mouth open. This is because a swallowing action begins with a change in pressure in the oral cavity, when the individual concerned moves his/her tongue forwards against the lower teeth, and then widens the oral cavity at the end of said action to create a partial vacuum which draws saliva located around the teeth into the oral cavity, whereafter the tongue is moved upwards against the front teeth to hard palate, whereupon the saliva collects on top of the tongue. This is a series of muscle movements which can be initiated by a natural desire, but which is normally initiated instinctively and automatically without the individual being conscious of the fact. The aforesaid partial vacuum cannot be established when the mouth is open and it is therefore not possible to swallow, which is why dentists are forced to use some form of suction device to remove saliva from the mouth of a patient.

Part function 2 takes as its starting point the fact that saliva is entrapped between the tongue and the teeth. When the tongue is drawn rapidly upwards the pressure against the soft palate and the throat increases. This part function can also be influenced by the will, although it is difficult to initiate when no saliva from the first part function is enclosed above the tongue.

A swallowing action can be interrupted by opening the passage to the forward oral cavity, which is what takes place when a dentist inserts an instrument into the mouth of a patient. When the swallowing action is continued in the second part function, the subsequent part functions are effected automatically, this being the case when the saliva passes the throat.

As a result of the invention, the instrument or object can be dislodged temporarily from the oral cavity without the use of the hands or other external means. Because of the particular positioning of the gripping surfaces on the instrument it can be moved outwardly of the mouth without falling therefrom, while leaving between the teeth a space of such small magnitude as to enable the necessary partial vacuum to be established for a normal swallowing action to take place, without undue effort on the part of the individual concerned.

When holding a relatively large object in the mouth at a fixed distance between the teeth, it is practically impossible to speak in an articulate fashion. If, on the other hand, the teeth can be closed or partially closed it is then possible to form words in a manner which can be understood. Thus, in the case of large objects held fully or partially in the mouth it is necessary to provide the objects with means which enables the teeth to be closed or partially closed, to facilitate speech.

### Brief Description of the Drawings

So that the invention will be more readily understood and further features thereof made apparent an embodiment illustrative of the invention will now be described with reference to the accompanying drawings. The embodiment selected by way of example to illustrate the principle of the invention relates to a heat-exchange device intended to improve the well-being of an individual and his/her physical working capacity, by recovering energy or adjusting the temperature of the inspired air at temperatures deviating from body temperature. It will be understood, however, that the use of other objects, instruments and devices, such as certain breathing devices, can be improved advantageously by applying the concept of the present invention.

In the drawings:
- Figure 1: illustrates in perspective an embodiment of the preferred arrangement applied to a heat-exchange device;
- Figure 2: illustrates the device of Figure 1 seen from one end;
- Figure 3: illustrates schematically an embodiment of the novel arrangement applied to the device chosen by way of example.

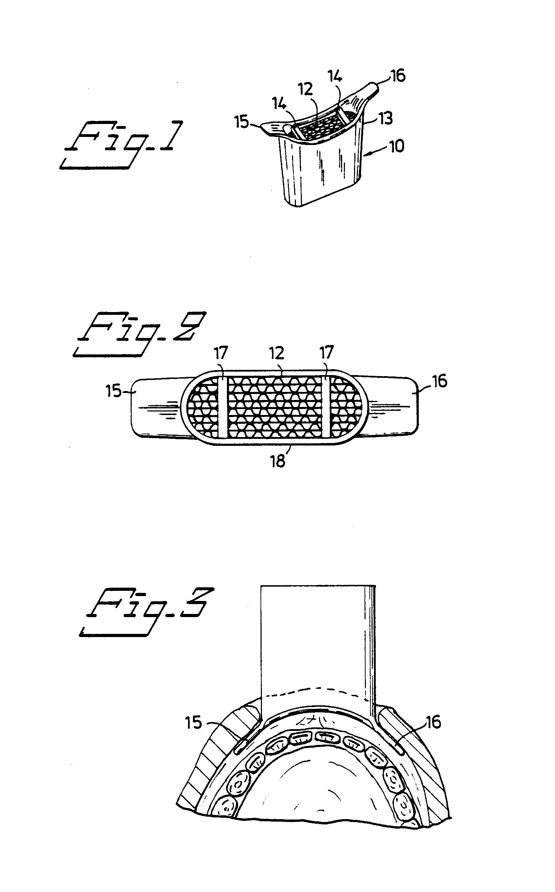

Figure 1 illustrates an energy recovering device 10 which comprises aluminium foil. One end of the device is of an undulating configuration and the other is planar or substantially planar. The foil is wound so as to form a large number of channels 12. These channels are open in opposite directions, such as to form an elongated rectangular heat-exchange body. This body is intended to be enclosed in a tubular casing 13 made of a soft, relatively soft or hard resilient material. The casing 13 is open at both ends thereof, the ends of the casing of the illustrated embodiment extending beyond respective end surfaces of the heat-exchanger body. Located at one open end of the casing 13 are one or two, preferably two, narrow transverse ribs 14 which join the side walls of the casing together. In the illustrated embodiment the ribs 14 are permanently connected to the casing. The casing side-walls at the other open end of the casing are inclined upwardly in mutually opposite directions from the centre and the side walls are joined and transform into outwardly jutting gripping surfaces, such as flanges 15 and 16 which extend in mutually opposite directions. The side walls of this other end are connected together by means of one or two, preferably two, mutually spaced ribs 17. In the illustrated embodiment these ribs 17 are permanently connected to the side-walls located between the ribs 17 are reinforced with a bead 18. As will be understood, the ribs 14 and/or 17 may be detachably connected to respective side walls, so as to enable both the casing and the heat-exchanger body to be changed. It will also be understood that the heat-exchanger body and the casing 13 may be held together in a manner different to that illustrated, so as to render the ribs 14 and 17 and the reinforcing bead 18 unnecessary. The angle defined by the flanges 15 and 16 with the casing 13 is suitably between 30° and 150°.

When wishing to use the illustrated heat-exchange device 17 to facilitate breathing during some form of physical work for example, it is inserted into the mouth with the gripping surfaces facing inwardly, where the device is retained directly or indirectly by the muscles of the oral cavity. The device is positioned in the mouth such that the gripping surfaces are located relatively far back in the oral cavity. Due to the resiliency of the material and/or the shape and size of the gripping surfaces they cause no discomfort to the wearer. When fitted, the device functions in a known manner as an energy-recovering device, i.e. there is obtained a reduction in the dead space in the oral cavity, increased breathing through the mouth and corresponing reduction in nostril ventilation, and recovery of the moisture and heat in the expired air. A known device of this kind is illustrated and described in WO-A- 84/01720. When the person wearing the device wishes to swallow or to speak without interrupting his/her work, the heat-exchanger device is moved out of the mouth with the tongue and/or with the aid of the muscles of the oral cavity to a position in which the device is retained in the oral cavity solely though or substantially through the support afforded by the flanges or wings 15, 16. When the device occupies this position, the easing 13 and the heat-exchanger body encased therein are located at a distance from the upper and lower teeth of a person wearing the device, so as to enable the teeth to be brought together and the person to swallow in a normal manner and to facilitate speech. The device is then returned to its operative position with the aid of the muscles of the oval cavity and alternatively also the teeth.

It will be understood that although the invention has been described in respect of a heat-exchanger device for improving the well-being and/or physical working ability of an individual, the means for enabling the position of the device in the mouth to be changed solely by the muscles of the oral cavity with optional assistance from the tongue and teeth can be applied equally as well to other objects intended to be held partially or wholly in the mouth.

It will also be understood that the flanges or wings 15, 16 need not form an integral part of the casing 13, but may be connected detachably thereto.

## Claims

1. Breathing device (10) which is intended to be placed and held in the mouth of a person and which comprises a tubular casing (13) and positioned therein a plurality of parallel channels (12) open at each end, the casing having a proximal open end opposite to a distal open end, the proximal end of the casing being concave and being provided thereon with two outwardly and laterally jutting gripping flanges (15, 16) extending in opposite directions, the gripping flanges extending at such an angle from the casing and being so dimensioned as:
- firstly to enable the device to be moved by the facial muscles and/or the tongue from an operative position, in which the flanges are located inwardly of the jaws or teeth relatively far back in the oral cavity, to a speaking or swallowing position, in which the device is held forwardly of the jaws or teeth, and to be returned to the operative position by the facial muscles and alternatively also the teeth;
- secondly to enable the person to hold the device firmly in the speaking or swallowing position by an interaction between the gripping flanges and the soft parts of the mouth, such as the cheeks or lips;
- and thirdly to permit the jaws to be substantially closed when the device is in the speaking or swallowing position.

2. Device according to claim 1, characterized in that it has the form of a heat-exchanging device.

3. Device according to claim 1 or claim 2, characterized in that the gripping flanges are permanently connected to the the casing.

4. Device according to claim 1 or claim 2, characterized in that the gripping flanges are detachably connected to the casing.

5. Device according to clamis 1, 2, 3 or 4, characterized in that the gripping flanges of the proximal end of the casing form an angle thereon of between 30° and 150°.

## Patentansprüche

1. Atmungsvorrichtung (10), die in dem Mund einer Person angeordnet und gehalten werden kann, welche ein rohrförmiges Gehäuse (13) mit einer Vielzahl von darin befindlichen parallelen Kanälen (12) unfaßt, die an jedem Ende offen sind, wobei das Gehäuse ein proximales offenes Ende und gegenüberliegend ein distales offenens Ende aufweist, wobei das proximale Ende des Gehäuses konkav ist und mit zwei auswärts und seitlich abragenden Greifflanschen (15, 16) versehen ist, die sich in entgegengesetzte Richtungen erstrecken, wobei sich die Greifflansche in einem solchen Winkel von dem Gehäuse erstrecken und derart bemessen sind, daß
erstens die Vorrichtung von den Gesichtsmuskeln und/oder der Zunge aus einer Betriebslage, in welcher die Flansche hinter den Kiefern oder Zähnen relativ weit hinten in dem Mundraum angeordnet sind, in eine Sprech- oder Schlucklage bewegbar ist, in welcher die Vorrichtung vor den Kiefern oder Zähnen gehalten ist, und sie in die Betriebslage durch die Gesichtsmuskeln und alternativ auch die Zähne zurückbewegbar ist,
zweitens die Person die Vorrichtung fest in der Sprech- oder Schlucklage durch Wechselwirkung zwischen den Greifflanschen und den Weichteilen des Mundes wie beispielsweise den Wangen oder Lippen halten kann und
drittens die Kiefer im wesentlichen eng anliegen können, wenn sich die Vorrichtung in der Sprech- oder Schlucklage befindet.

2. Vorrichtung nach Anspruch 1, dadurch gekennzeichnet, daß sie die Form einer Wärmeaustauschvorrichtung hat.

3. Vorrichtung nach Anspruch 1 oder 2, dadurch gekennzeichnet, daß die Greifflansche dauernd mit dem Gehäuse verbunden sind.

4. Vorrichtung nach Anspruch 1 oder 2, dadurch gekennzeichnet, daß die Greifflansche lösbar mit dem Gehäuse verbunden sind.

5. Vorrichtung nach Auspruch 1, 2, 3 oder 4, dadurch gekennzeichnet, daß die Greifflansche an dem proximalen Ende des Gehäuses einen Winkel von zwischen 30° und 150° bilden.

## Revendications

1. Appareil respiratoire (10) destiné à être placé et tenu dans la bouche d'un utilisateur et comprenant une enveloppe tubulaire (13) et, prévus là-dedans, une pluralité de canaux parallèles (12) ouverts à chaque extrémité, ladite enveloppe ayant une extrémité ouverte proximale qui est opposée à une extrémité ouverte distale, ladite extrémité proximale de l'enveloppe étant concave et pourvue de deux ailes de prise (15, 16) faisant saillie latéralement vers l'extérieur et s'étendant dans des directions opposées, les ailes de prise s'étendant depuis l'enveloppe sous un tel angle et étant dimensionnées de telle manière:
- qu'en premier lieu, elles permettent à l'appareil d'être déplacé par les muscles faciaux et/ou la langue d'une position active, dans laquelle les ailes se trouvent derrière les mâchoires ou les dents relativement loin en arrière dans la cavité buccale, jusqu'à une position de discours ou d'avalement, dans laquelle l'appareil est tenu en avant des mâchoires ou des dents, et d'être ramené à ladite position active par les muscles faciaux ou bien par les dents aussi;
- qu'en deuxième lieu, elles permettent à l'utilisateur de tenir l'appareil fermement dans la position de discours ou d'avalement grâce à une interaction entre les ailes de prise et les parties molles de la bouche, telles que les joues et les lèvres; et
- qu'en troisième lieu, elles permettent aux mâchoires d'être sensiblement fermées lorsque l'appareil est dans la position de discours ou d'avalement.

2. Appareil selon la revendication 1, **caractérisé** en ce qu'il est un dispositif échangeur de chaleur.

3. Appareil selon la revendication 1 ou 2, **caractérisé** en ce que les ailes de prise sont reliées à demeure à l'enveloppe.

4. Appareil selon la revendication 1 ou 2, **caractérisé** en ce que les ailes de prise sont reliées de façon amovible à l'enveloppe.

5. Appareil selon la revendication 1, 2, 3 ou 4, **caractérisé** en ce que les ailes de prise à l'extrémité proximale de l'enveloppe y forment un angle compris entre 30° et 150°.
